# EUROPÄISCHE PATENTSCHRIFT

(11) **EP 3 724 625 B1**
(45) Veröffentlichungstag und Bekanntmachung des Hinweises auf die Patenterteilung: **31.01.2024**
(21) Anmeldenummer: 18819084.7
(22) Anmeldetag: 11.12.2018
(51) Int. Cl.: G01L 5/00, A61F 2/30

(54) **LINEARKRAFTMESSEINRICHTUNG UND HYDRAULIKAKTUATOR**
LINEAR FORCE-MEASURING DEVICE AND HYDRAULIC ACTUATOR
DISPOSITIF DE MESURE DE FORCE LINÉAIRE ET ACTIONNEUR HYDRAULIQUE

(30) Priorität: 11.12.2017 DE 102017129486
(43) Veröffentlichungstag der Anmeldung: 21.10.2020
(73) Patentinhaber: Otto Bock Healthcare Products GmbH, 1110 Wien (AT)
(72) Erfinder: AUBERGER, Roland, 1140 Wien (AT); BREUER-RUESCH, Christian, 1230 Wien (AT); GRADISCHAR, Andreas, 1170 Wien (AT); NINO, Juan Pablo Mejia, 1160 Wien (AT); SPRING, Alexander Noah, Ottawa, Ontario K1Y 1W3 (CA)
(74) Vertreter: Gramm, Lins & Partner Patent- und Rechtsanwälte PartGmbB
(86) Internationale Anmeldenummer: PCT/EP2018/084324
(87) Internationale Veröffentlichungsnummer: WO 2019/115513

(56) Entgegenhaltungen:
- EP-A1- 1 528 382
- EP-A1- 1 696 216
- EP-A1- 2 090 793
- EP-A2- 1 285 640
- WO-A1-02/02450
- WO-A1-94/07118
- WO-A1-2011/123928
- CN-A- 107 339 318
- CN-U- 202 614 432
- DE-A1- 19 917 020

## Beschreibung

Die Erfindung betrifft eine Linearkraftmesseinrichtung für einen Hydraulikaktuator einer orthopädietechnischen Einrichtung mit einer Basis, einer Lageraufnahme und zumindest einem Sensor zur Erfassung von Längenänderungen zwischen der Basis und der Lageraufnahme sowie einen Hydraulikaktuator mit einer solchen Linearkraftmesseinrichtung.

Bei orthopädietechnischen Einrichtungen wie Orthesen, Prothesen oder auf Rollstühlen ist es zur Bestimmung von Belastungen, Lastwechselzyklen oder zur Steuerung von Hydraulikaktuatoren wie Hydraulikdämpfern und/oder hydraulischen Antrieben notwendig, Sensordaten zur Verfügung zu stellen, auf deren Grundlage beispielsweise Antriebe aktiviert oder Ventile verstellt, Bewegungsmuster erfasst oder Bauteilbelastungen abgeschätzt werden. Belastungen können beispielsweise über Drucksensoren oder Momentensensoren aufgenommen werden.

Eine weitere Möglichkeit zur Messung von Kräften aufgrund von Längenänderungen besteht in der Anordnung von Dehnmessstreifen zur Erfassung von Dehnungen oder Stauchungen. Zur Erfassung von Kräften, die auf einen Linearaktuator wirken oder von einem Linearaktuator ausgeübt werden, werden Dehnmessstreifen an dem Linearaktuator befestigt. Problematisch bei der Erfassung der Kräfte ist dabei, dass unterschiedliche Einbaulagen bei gleicher Belastung zu unterschiedlichen Messwerten führen können. Darüber hinaus ist ein modularer Aufbau nicht gegeben und die Signalqualität ist vielfach nicht sehr hoch.

Die EP 1 528 882 A1 betrifft eine Kombination aus einem Lagerblock mit Querstreben in der Basis und einer unterhalb der Basis angeordneten Messplatte mit Dehnmessstreifen.

Die EP 1 696 216 A1 betrifft ein Verfahren und eine Vorrichtung zum Messen eines Drehmomentes in einem Handhabungsgerät, bei dem eine Wellenlagerung sich über einen Sensor oder mehrere Sensoren an einer Komponente des Handhabungsgerätes abstützt.

Die WO 94/07118 A1 betrifft eine Einrichtung zur Erfassung radialer Kräfte mit einem Innenring zur Aufnahme einer Welle und einem äußeren Ring, dessen innerer Durchmesser größer als der äußere Durchmesser des inneren Ringes ist. Die beiden Ringe sind mit zwei einander gegenüberliegende Speichen miteinander verbunden, an den Speichen sind Messbereiche angeordnet, die zur Erfassung radialer Kräfte mit magnetoelastischen Messwertaufnehmern ausgebildet sind. CN 107339318 A und EP 1528382 A1 offenbaren Kraftmesseinrichtungen mit Lageraufnahme, Stegen, Versteifungselement und Basis.

Aufgabe der vorliegenden Erfindung ist es daher, eine Linearkraftmesseinrichtung bereitzustellen, die eine hohe Signalqualität bei unterschiedlichen Anwendungen sowie Einbausituationen eines Hydraulikaktuators bereitstellt.

Erfindungsgemäß wird diese Aufgabe durch eine Linearkraftmesseinrichtung mit den Merkmalen des Hauptanspruches und einen Hydraulikaktuator mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Die Linearkraftmesseinrichtung für einen Hydraulikaktuator einer orthopädietechnischen Einrichtung mit einer Basis, einer Lageraufnahme und zumindest einem Sensor zur Erfassung von Längenänderungen zwischen der Basis und der Lageraufnahme sieht vor, dass die Lageraufnahme mit der Basis über zwei einander gegenüberliegende Stege stoffschlüssig verbunden ist und dass der zumindest eine Sensor an einem der Stege befestigt ist. Durch die Verbindung einer Lageraufnahme, die zu der Befestigung eines Hydraulikaktuators an einer weiteren Komponente der orthopädietechnischen Einrichtung dient, beispielsweise einer Prothese oder Orthese, mit einer Basis, über die die Linearkraftmesseinrichtung mit dem Hydraulikaktuator gekoppelt werden kann, ist ein kostengünstiger, modularer Aufbau orthopädietechnischer Komponenten möglich. Über die Stege kann ausreichend Platz für die Befestigung eines Sensors oder mehrerer Sensoren bereitgestellt werden. Die Anordnung des Sensors an den Stegen ermöglicht eine optimierte Befestigung und gewährleistet eine hohe Signalqualität. Durch die Verbindung der Lageraufnahme mit der Basis über zwei Stege wird eine symmetrische Kraftdurchleitung bewirkt, so dass bei der Aufbringung von Zugkräften oder Druckkräften zwischen der Lageraufnahme und der Basis diese Kräfte gleichmäßig weitergeleitet werden. Dadurch werden Biegemomente verringert oder ausgeschlossen, die eine Kraftmessung beeinflussen können. Darüber hinaus wird eine hohe Steifigkeit durch die beiden zueinander beabstandeten, einander gegenüberliegenden, bevorzugt parallel zueinander ausgerichteten Stege erreicht. Die Leistungsmöglichkeiten des DMS werden dadurch optimal ausgenutzt. Der Sensor oder die Sensoren sind insbesondere als Dehnmessstreifen oder Piezoelemente ausgebildet. Die Erfindung sieht vor, dass zwischen den Stegen ein Versteifungselement angeordnet ist. Durch das Versteifungselement, das zwischen den Stegen angeordnet ist, wird eine weitere Versteifung der Stege bewirkt. Dadurch werden bei dem Aufbringen von Zugkräften oder Druckkräften Biegeeffekte oder Knickneigungen eliminiert, so dass sich eine sehr lineare Kennlinie sowohl in Zugrichtung als auch in Druckrichtung erzielen lässt. Durch die Basis, die Stege und die Lageraufnahme in Verbindung mit dem Versteifungselement wird ein Aufnahmeraum ausgebildet, insbesondere wenn das Versteifungselement sowohl an der Basis als auch an den Stegen und der Lageraufnahme anliegt. Innerhalb des Aufnahmeraumes, der einseitig offen ausgebildet ist, kann eine Steuerungselektronik oder eine Auswerteelektronik angeordnet sein, die mit dem Sensor, z.B. Dehnmessstreifen oder Piezoelement, verbunden ist, vorzugsweise über eine Kabelverbindung, insbesondere ein Flachbandkabel.

Die Stege sind als Wände ausgebildet, wodurch sich bei einem geringen Gewicht eine maximale Steifigkeit erreichen lässt, ohne dass die Signalstärke des Sensors verringert wird. Bevorzugt sind die Stege trapezförmig ausgebildet und verjüngen sich in Richtung auf die Lageraufnahme, so dass sich bei einem gleichschenkeligen Trapez die längere der beiden parallelen Seiten an der Basis befindet, während sich die kürzere der parallelen Seiten an der Lageraufnahme befindet. Bevorzugt ist die Symmetrieachse der trapezförmigen Wand mittig an der Lageraufnahme angeordnet, so dass die Kräfte, die über die Lageraufnahme in die Linearkraftmesseinrichtung eingeleitet werden, gleichmäßig auf die Stege verteilt werden und eine gleichmäßige Längung oder Stauchung auswirken.

Die Stege können eine gleichmäßige Wandstärke aufweisen, um ein homogenes Spannungsfeld aufzubauen und ein präzises Signal über den Sensor, z.B. Dehnmessstreifen oder Piezoelement zu erhalten.

Bevorzugt sind die Stege parallel zu der Kraftwirkungsrichtung ausgerichtet, das heißt, dass sie sich in Kraftwirkungsrichtung der Linearkraft, die von der Lageraufnahme auf die Basis und zurück wirkt, von der Basis zu der Lageraufnahme erstrecken. Die Stege sind bevorzugt parallel zueinander orientiert, um Biegemomente und Querkräfte, die bei einer schrägen Ausgestaltung der Stege relativ zu der Kraftwirkungsrichtung auftreten könnten, möglichst zu vermeiden. Grundsätzlich ist es auch möglich, eine geringe Neigung der Stege relativ zu der Kraftwirkungsrichtung zu erlauben.

In einer Weiterbildung der Erfindung ist es vorgesehen, dass die Basis als eine runde Scheibe ausgebildet ist, auf der die Linearkraftmesseinrichtung vollflächig oder zumindest vollumfänglich auf einer entsprechenden Aufnahme, beispielsweise eines Hydraulikaktuators, aufliegt. Dadurch ist es möglich, die Linearkraftmesseinrichtung drehbar an dem jeweiligen Objekt zu befestigen, so dass eine gewünschte Orientierung der Linearkraftmesseinrichtung relativ zu der Befestigungsstelle an dem lageraufnahmeseitigen Ende oder dem basisseitigen Ende erfolgen kann. Die Basis kann auch zumindest einen Absatz oder einen umlaufenden Vorsprung aufweisen, über den die Basis an oder in einer Aufnahme einer weiteren Komponente zentriert oder ausgerichtet werden kann.

Die Lageraufnahme ist bevorzugt hülsenförmig mit einer runden Ausnehmung ausgebildet, um die Lageraufnahme mit einem Lager an entsprechenden Befestigungselementen wie Bolzen, Zapfen, einem Befestigungsrahmen oder dergleichen festlegen zu können. Durch die runde Ausgestaltung der Ausnehmung ist es möglich, die üblichen Lager, insbesondere Pendellager wie Pendelrollenlager oder Pendelgleitlager oder auch Kugelgelenklager, in der Lageraufnahme anzuordnen, so dass eine weitgehende Verstellmöglichkeit der Linearkraftmesseinrichtung relativ zu der Lagerstelle erreicht werden kann, ohne dass Querkräfte, Verdrehungen oder Verspannungen innerhalb der Linearkraftmesseinrichtung auftreten. Darüber hinaus kann durch die Verdrehbarkeit um zumindest zwei rotatorische Freiheitsgrade eine rechtsseitige oder linksseitige Anwendung bei lateralen Anordnungen an orthopädietechnischen Einrichtungen wie insbesondere Orthesen oder Prothesen erfolgen, so dass sich eine weitgehende Spannungsentkopplung in der Linearkraftmesseinrichtung einstellt. Dies führt dazu, dass nur die Linearkräfte, die zwischen der Lageraufnahme und der Basis in Richtung aufeinander zu oder voneinander weg auftreten, über den Sensor, oder die Sensoren, z.B. den Dehnmessstreifen oder die Dehnmessstreifen gemessen werden.

Die Basis, die Stege und die Lageraufnahme können einstückig ausgebildet sein, beispielsweise gesintert oder gegossen oder in einem anderen Urformverfahren hergestellt sein. Dadurch wird der Fertigungsaufwand verringert und eine gleichmäßige Krafteinleitung und -durchleitung von der Basis in die Lageraufnahme erreicht.

Das Versteifungselement ist als eine Wand ausgebildet, die zwischen den Stegen verläuft. Die Stege sind bevorzugt parallel zueinander ausgerichtet, so dass sich die Wand als Versteifungselement senkrecht zu dem jeweiligen Steg erstreckt. Die Wand erstreckt sich über den gesamten Bereich zwischen der Basis und der Lageraufnahme, so dass sie sowohl mit der Lageraufnahme als auch mit der Basis verbunden ist. Die Stege sind zudem mit dem Versteifungselement verbunden, so dass sich insbesondere bei einer mittigen Anordnung des Versteifungselementes im Bereich der Ebene, die entlang der Symmetrie der trapezförmigen Stege angeordnet ist und sich senkrecht davon in Richtung auf den gegenüberliegenden Steg erstreckt, eine symmetrische Ausgestaltung der Stege und des Versteifungselementes ergibt. Die Kontur des Versteifungselementes zusammen mit den beiden Stegen entspricht oder ähnelt dabei einem I-Träger oder einem H-Träger, wodurch sich eine hohe Steifigkeit bei einem geringen Materialeinsatz und damit eine kompakte Bauweise mit geringem Gewicht erreichen lässt. Das Versteifungselement in Gestalt einer Wand verhindert wirksam eine Biegung der Stege, insbesondere bei einer Stauchung der Stege. Das Versteifungselement ist bevorzugt mittig zu der Lageraufnahme angeordnet, so dass das Versteifungselement in einer Symmetrieebene für die Linearmesskrafteinrichtung liegt.

Bei einer symmetrischen Ausgestaltung des Versteifungselementes, bei einer vollständigen Anlage an der Basis, den Stegen und der Lageraufnahme, werden zwei Aufnahmeräume beiderseits des Versteifungselementes oder der Versteifungswand gebildet, so dass auf einander gegenüberliegenden Seiten des Versteifungselementes ausreichend Raum durch die Stege, die Lageraufnahme und die Basis gebildet wird, um beispielsweise eine zweite Steuerungseinrichtung für einen zweiten Sensor darin anzuordnen. Ebenfalls können Sendeeinrichtungen oder Anschlüsse in dem Aufnahmeraum angeordnet sein. Sowohl der Sensor oder die Sensoren und die Auswerte- und/oder Steuerungselektronik können in einem Kunststoff eingegossen und davon ummantelt sein, um einen mechanischen Schutz der Elektronik zu erreichen. Bevorzugt ist der Sensor an der Außenseite des Steges oder eines Stege angeordnet, um die dort zur Verfügung stehende, bevorzugt ebene Fläche auszunutzen.

Der erfindungsgemäße Hydraulikaktuator, der beispielsweise als Hydraulikdämpfer oder als hydraulischer Antrieb oder eine Kombination davon ausgebildet sein kann, der für eine orthopädietechnische Einrichtung mit einer Kolben-Zylindereinheit ausgestattet ist und eine Aufnahme aufweist, an der oder in der eine Linearkraftmesseinrichtung wie oben beschrieben, angeordnet ist, hat den Vorteil, dass mit einer hohen Steifigkeit Kräfte über die Linearkraftmesseinrichtung von einer Lagerstelle des Hydraulikaktuators in den Hydraulikaktuator eingeleitet werden kann, beispielsweise um Kräfte von einem Prothesen- oder Orthesenoberteil an ein Prothesen- oder Orthesenunterteil weiterzuleiten oder aufzunehmen. Gleichzeitig können die dort auftretenden Zug- oder Druckkräfte über den Sensor gemessen und zur Steuerung des Hydraulikaktuators verwendet werden. Dazu werden die gemessenen Kräfte von der Auswerte- und/oder Steuerungselektronik ausgewertet und in Steuerungssignale umgewandelt, über die entweder Antriebe aktiviert oder deaktiviert werden, um die Kolben-Zylindereinheit relativ zueinander zu bewegen oder um Ventile zu öffnen oder zu schließen. Durch die Befestigung der Linearkraftmesseinrichtung an oder in einer Aufnahme ist es möglich, unterschiedliche Hydraulikkomponenten mit der Linearkraftmesseinrichtung zu koppeln. Darüber hinaus ist es möglich, eine Messeinrichtung gleichzeitig mit einer Anbindung der Hydraulik an die orthopädietechnische Komponente zu koppeln, so dass die Linearkraftmesseinrichtung gleichzeitig die Verbindung zwischen der orthopädietechnischen Einrichtung und der Hydraulikkomponente bildet. Durch die Anordnung der Linearkraftmesseinrichtung in der Aufnahme des Hydraulikaktuators kann die Kraftmessung im Wesentlichen unabhängig von Biegemomenten innerhalb der Hydraulikeinheit erfolgen.

Eine Weiterbildung der Erfindung sieht vor, dass die Basis der Linearkraftmesseinrichtung vollflächig oder zumindest vollumfänglich auf der Aufnahme aufliegt. Insbesondere in Verbindung mit einer runden Ausgestaltung der Basis und einer entsprechend runden Aufnahme ist es möglich, die Linearkraftmesseinrichtung verdrehbar an dem Hydraulikaktuator anzuordnen. Durch die vollflächige oder zumindest vollumfängliche Auflage der Basis auf der Aufnahme oder in der Aufnahme ist es möglich, unabhängig von der Orientierung der Linearkraftmesseinrichtung eine gleichmäßige Krafteinleitung in die Basis zu erreichen. Dadurch wird die durchzuleitende Kraft von der Basis vollständig auf die Stege übertragen. Eine Verbiegung oder eine punktuelle Krafteinleitung wird reduziert oder ausgeschlossen.

Bei einer drehbaren Lagerung der Basis auf oder in der Aufnahme ist die Basis vorteilhafterweise klemmend an der Aufnahme fixiert. Diese Klemmung kann beispielsweise über eine Überwurfmutter erfolgen, die auf ein Außengewinde an der Aufnahme aufgeschraubt wird und die Basis gleichmäßig in Richtung auf die Aufnahme drückt und daran festklemmt.

In der Lageraufnahme ist bevorzugt ein Pendellager oder ein Kugelgelenk angeordnet, so dass der gesamte Hydraulikaktuator in zumindest zwei rotatorischen Freiheitsgraden relativ zu der orthopädietechnischen Einrichtung verlagert werden kann. Daraus ergibt sich eine nahezu spannungsfreie Krafteinleitung von den Befestigungseinrichtungen der orthopädietechnischen Einrichtung in den Hydraulikaktuator über die Linearkraftmesseinrichtung. Die Aufnahme kann daher beispielsweise an einem Gehäuse des Hydraulikaktuators oder einer Kolbenstange angeordnet oder ausgebildet sein, so dass die klemmende Festlegung über die Überwurfmutter oder über eine andere Klemmeinrichtung an einer nahezu beliebigen Stelle des Hydraulikaktuators erfolgen kann. Die Positionierung entweder an dem Gehäuse oder einer Kolbenstange erfolgt in Abhängigkeit von den geometrischen Gegebenheiten und Notwendigkeiten der orthopädietechnischen Einrichtung.

Mit der erfindungsgemäßen Lösung ist es möglich, eine positionsunabhängige Befestigung des Sensors, z.B. des Dehnmessstreifens oder Piezoelementes, als Kraftsensor in Bezug auf den Hydraulikaktuator zu erreichen, insbesondere unabhängig in Bezug auf das Gehäuse oder eine Kolbenstange hinsichtlich der Längserstreckung oder der Kraftwirkungslinie. Über die Linearkraftmesseinrichtung wird ein Sensorträger, insbesondere Dehnmessstreifenträger oder Piezoelemententräger bereitgestellt, der zudem die Anbindung eines Hydraulikaktuators an der orthopädietechnischen Einrichtung definiert. Die Linearkraftmesseinrichtung ist zu der Hydraulikeinheit verstellbar, insbesondere verdrehbar angeordnet, so dass durch Verwendung der modularen Baugruppen beispielsweise ein Hydraulikaktuator linksseitig oder rechtsseitig lateral an einer Orthese oder Prothese angeordnet werden kann.

Durch die Geometrie der Linearkraftmesseinrichtung ergibt sich ein homogenes Spannungsfeld bei einem kleinen Bauvolumen mit sehr hoher Festigkeit. Daraus kann eine lineare Kennlinie in Zug- und Druckrichtung mit einer hohen Signalqualität erzielt werden.

Nachfolgend wird ein Ausführungsbeispiel der Erfindung anhand der beigefügten Figuren näher erläutert. Es zeigen:
- Figur 1 -: eine perspektivische Darstellung einer Linearkraftmesseinrichtung ohne DMS;
- Figur 2 -: eine Explosionsdarstellung einer Linearkraftmesseinrichtung; sowie
- Figur 3 -: einen Hydraulikaktuator mit montierter Linearkraftmesseinrichtung,
- Figur 4 -: eine Schnittdarstellung eines Hydraulikaktuators gemäß Figur 3;
- Figur 5 -: eine Vertikalschnittdarstellung einer Linearkraftmesseinrichtung;
- Figur 6 -: eine Seitenansicht einer Linearkraftmesseinrichtung;
- Figur 7 -: ein Anwendungsbeispiel an einer Orthese; sowie
- Figur 8 -: eine Anwendung eines Hydraulikaktuators mit einer Linearkraftmesseinrichtung in einer Prothese

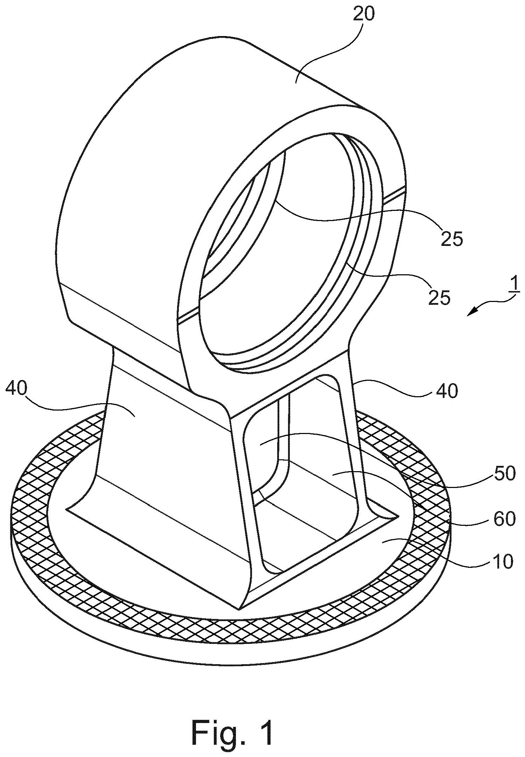

In der Figur 1 ist in einer perspektivischen Darstellung eine Linearkraftmesseinrichtung 1 für einen Hydraulikaktuator einer orthopädietechnischen Einrichtung wie beispielsweise einer Orthese, Exoskelett, Prothese oder Rollstuhl gezeigt, mit einer Basis 10, die in dem dargestellten Ausführungsbeispiel scheibenförmig und rund ausgebildet ist. Auf einer Oberseite der Basis 10 ist eine Lageraufnahme 20 über zwei Stege 40 mit der Basis 10 verbunden. Die Basis 10, die Lageraufnahme 20 und die Stege 40 sind in dem dargestellten Ausführungsbeispiel einstückig hergestellt, beispielsweise gesenkgeschmiedet, gegossen, gesintert oder gepresst. In einer alternativen Ausführungsform der Erfindung kann die Basis 10 separat von der Lageraufnahme 20 und den Stegen 40 ausgebildet sein, wobei die Lageraufnahme 20 und die Stege 40 entweder einstückig ausgebildet oder aus separaten Komponenten gefertigt und stoffschlüssig miteinander verbunden sind. Neben der Herstellung in einem Urformverfahren können Basis 10, Lageraufnahme 20 und Stege 40 im Rahmen eines Trennverfahrens und/oder Umformverfahrens hergestellt werden. Werden separate Teile gefertigt, sind diese stoffschlüssig miteinander verbunden, insbesondere verschweißt. Sowohl die Basis 10 als auch die Stege 40 und die Lageraufnahme 20 sind bevorzugt aus einem Metall, insbesondere einem Leichtmetall oder einem anderen hochfesten Werkstoff hergestellt. Auch eine Fertigung über faserverstärkte Kunststoffe ist möglich. Die Basis 10 weist auf der der Lageraufnahme 20 abgewandten Unterseite in einer Ausführungsform einen umlaufenden Absatz auf, so dass sich auf der Unterseite ein erhabener, kreisförmiger Sockel ergibt. Darüber ist es möglich, dass die Basis 10 vollumfänglich auf einer ringförmigen Auflage an oder in einer Aufnahme eines Hydraulikaktuators aufliegt. Darüber hinaus wird über den Sockel die Basis 10 innerhalb der Aufnahme zentriert. Alternativ zu einem Absatz oder einem kreisförmigen Vorsprung ist es möglich, die Unterseite der Basis 10 eben oder glatt auszugestalten, so dass eine vollflächige Auflage auf einer korrespondierend ausgebildeten Oberfläche der Aufnahme möglich ist.

Die beiden Stege 40 sind parallel zueinander orientiert und können sich von der Basis 10 weg in Richtung auf die Lageraufnahme 20 entweder ebenfalls parallel erstrecken oder eine leichte Neigung zueinander aufweisen. Bei einer parallelen Orientierung der Stege 40 zueinander und einer im Wesentlichen orthogonalen Orientierung der Stege 40 von der Basis 10 weg in Richtung auf die Lageraufnahme 20 werden Zugkräfte und Druckkräfte, die von der Lageraufnahme 20 über die Stege 40 auf die Basis 10 übertragen werden, geradlinig durch die Stege 40 hindurchgeleitet, so dass sich eine nahezu ideale Längung oder Stauchung der Stege 40 bei einer entsprechenden Belastung ergibt. An der Außenseite der Stege 40 kann dann zumindest ein Dehnmessstreifen oder können mehrere Dehnmessstreifen angeordnet werden, um über die Messung von Längenänderungen Zugkräfte und Druckkräfte zu erfassen, die über die Lageraufnahme 10 durch die Stege 40 in die Basis 20 übertragen werden.

Die Lageraufnahme 20 ist hülsenförmig ausgebildet und weist auf der Innenseite ihrer Endbereiche Nuten 25 auf, in denen Federringe oder andere Sicherungsscheiben oder Sicherungselemente eingesetzt werden können, um einen in der Figur 1 nicht dargestellten Lagerkörper, beispielsweise Gleitlagerkörper oder Rollenlagerkörper, gegen eine Axialverlagerung aus der Lageraufnahme 20 zu sichern.

Die Lageraufnahme 20 sitzt auf den beiden Stegen 40 und bevorzugt mittig auf der Basis 10, so dass sich ein klappsymmetrischer Aufbau sowohl zu einer Ebene senkrecht zu der Durchgangsachse durch die Ausnehmung durch die Lageraufnahme 20 und orthogonal zu der Basis 10 sowie ebenso eine klappsymmetrische Ebene orthogonal zu der Basis 10 und parallel zu der Erstreckung der Stege 40 ergibt, wobei in der zweiten Klappsymmetrieebene die fiktive Achse der Ausnehmung der Lageraufnahme 20 verläuft.

Zwischen den Stegen 40 ist ein Versteifungselement 50 angeordnet, das als eine Wand ausgebildet ist, die sich zwischen den Innenseiten, also den beiden einander zugewandten Seiten, der Stege 40 erstreckt. Die Wand als Versteifungselement 50 erstreckt sich über die gesamte Breite des Zwischenraumes zwischen den beiden Stegen 40 und ist mit den Stegen 40 verbunden. Das Versteifungselement 50 kann separat eingesetzt und mit den Stegen 40, die als Wände ausgebildet sind, verklebt oder verschweißt oder verlötet sein. Alternativ kann die Wand 50 auch einstückig mit den Stegen 40 und/oder der Basis 10 und/oder der Lageraufnahme 20 ausgebildet sein. Die Wand 50 als Versteifungselement erstreckt sich im Wesentlichen orthogonal zu der Basis 10 und innerhalb der Klappsymmetrieebene und kann mit der Basis 10 verbunden sein, beispielsweise verklebt, verschweißt, verlötet oder auf eine andere Art und Weise. Ebenfalls kann das Versteifungselement 50 mit der Lageraufnahme 20 an dessen Außenseite oder Unterseite verbunden sein, insbesondere verklebt, verlötet, verschweißt oder auf eine andere Art und Weise. Eine wesentliche Aufgabe des Versteifungselementes 50 ist es, die Knickneigung der Stege 40 bei Aufbringen einer Drucklast sowie eine Neigung zur Biegung bei Aufbringung einer Zuglast zu verhindern, so dass eine verbesserte Signalqualität durch eine Längenänderung innerhalb der Stege bei Aufbringung einer Zuglast oder Drucklast erreicht werden kann.

Zwischen den Stegen 40 kann an der der Basis 10 zugewandten Grundseite ein Fußbereich ausgebildet sein, der verbreitert ausgebildet ist, so dass sich an dem Übergang der Stege 40 zu der Basis 10 eine Verbreiterung ergibt, so dass Kräfte, die durch die Stege 40 aufgebracht werden, gleichmäßig in die Basis 10 eingeleitet werden. Beiderseits des Versteifungselementes 50 wird durch die Stege 40, die Basis 10 oder den Fußbereich sowie die Lageraufnahme 20 ein Aufnahmeraum 60 abgegrenzt, der eine einseitige Öffnung aufweist, so dass in den Aufnahmeraum 60 elektrische oder elektronische Komponenten eingeführt werden können.

Die Stege 40 können trapezförmig ausgebildet sein, also sich von dem Fußbereich im Bereich der Basis 10 zum Kopfbereich im Bereich der Lageraufnahme 20 verjüngen, grundsätzlich ist es auch möglich, dass die Stege parallele Stirnseiten aufweisen, was insbesondere nur dann sinnvoll ist, wenn die Lageraufnahme 20 vergleichsweise bereit ist und einen Großteil der Fläche der Basis 10 abdeckt. Grundsätzlich ist es auch möglich und Gegenstand der Erfindung, wenn kein Versteifungselement 50 zwischen den Stegen angeordnet ist.

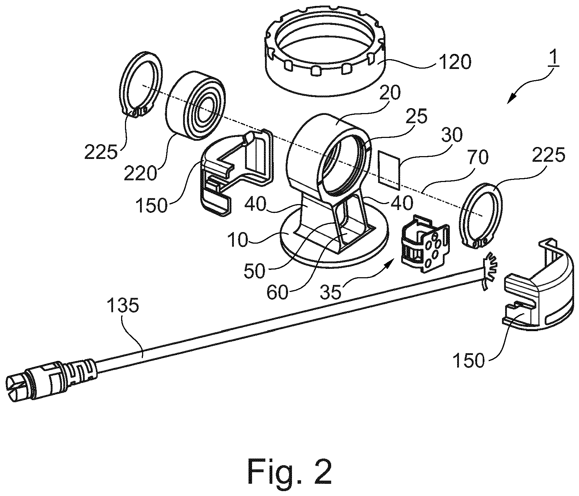

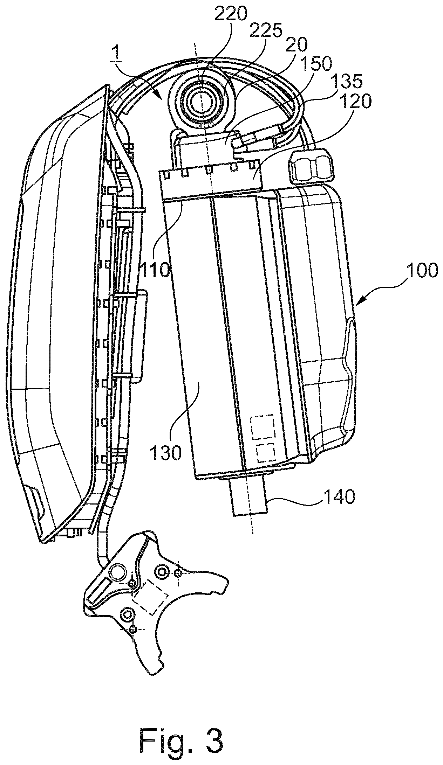

Figur 2 zeigt in einer Explosionsdarstellung die Linearkraftmesseinrichtung 1 mit der Basis 10, den beiden Stegen 40, der Lageraufnahme 20 sowie dem zwischen den Stegen 40 angeordneten Versteifungselement 50. Auch in diesem Ausführungsbeispiel ist die Basis 10 als kreisförmige Scheibe ausgebildet. Ein Sensor 30 in Gestalt eines Dehnmessstreifens 30 ist getrennt von der einen Außenseite eines Steges 40 dargestellt. Neben einem Dehnmessstreifen kann der Sensor 30 auch eine andere Bauform oder ein anderes Funktionsprinzip aufweisen, z.B als ein Piezoelement ausgebildet sein. Wird nachfolgend beispielhaft von einem Dehnmessstreifen 30 gesprochen, sind alle geeigneten Sensoren damit umfasst. Der Dehnmessstreifen 30 wird durch geeignete Maßnahmen auf der Außenseite des Steges 40 befestigt, beispielsweise aufgeklebt und mit einer elektronischen Auswerte- und/oder Steuerungseinrichtung 35 gekoppelt. Die elektronische Auswerte- und/oder Steuerungseinrichtung 35 ist so dimensioniert, dass sie in den Aufnahmeraum 60 eingebracht werden kann, der von der Basis 10, den Stegen 40, der Lageraufnahme 20 und dem Versteifungselement 50 begrenzt wird. An der Außenseite der Auswerte- und/oder Steuerungseinrichtung 35 können über fingerartige Kontaktanschlüsse und ein Kabel 135 die Messwerte des Dehnmessstreifens 30 zu einer weiteren Verwendung abgeleitet werden. Für die Lageraufnahme 20 ist ein Lagerkörper 220, der insbesondere als Pendellager, wie Pendelrollenlager oder Pendelgleitlager, oder als Kugelgelenk ausgebildet ist, vorgesehen. Der Lagerkörper 220 wird entlang der Bohrungsachse 70 in die Lageraufnahme 20 eingeführt und über Federringe 225, die in die korrespondierenden Nuten 25 innerhalb der Lageraufnahme 20 eingesetzt werden, gegen eine Verlagerung aus dem Lagerkörper 20 in Axialrichtung der Achse 70 gesichert. Nach Montage des Lagerkörpers 220 sowie der Anbringung des Dehnmessstreifens 30 und der Auswerte- und/oder Steuerungseinrichtung 35 wird die Linearkraftmesseinrichtung 1 über zwei Gehäusehälften 150 im Bereich der Stege 40 eingekapselt, um die Dehnmessstreifen 30 und die Auswerte- und/oder Steuerungseinrichtung 35 gegenüber äußeren Belastungen und Umwelteinflüssen zu schützen. Die Auswerte- und/oder Steuerungseinrichtung 35 sowie der Dehnmessstreifen 30 können auch von einem Kunststoff ummantelt und vergossen werden. Die Gehäusehälften 150 können dann nachträglich noch um den Bereich der Linearkraftmesseinrichtung 1 zwischen der Lageraufnahme 20 und der Basis 10 angeordnet werden. Die Gehäusehälften 150 sind so dimensioniert, dass sie radial nicht über die Basis 10 hinausragen, insbesondere wird ein Auflagerand von der Basis 10 bereitgehalten, um eine Überwurfmutter 120 anzubringen, mit der die Linearkraftmesseinrichtung 1 an einem Hydraulikaktuator befestigbar ist. Eine solche Ausgestaltung ist in der Figur 3 dargestellt.

In der Figur 3 ist in einer Seitenansicht ein Hydraulikaktuator 100 in Gestalt einer hydraulischen Dämpfereinrichtung gezeigt. Der Hydraulikaktuator 100 weist ein Gehäuse 130 auf, an dem eine Aufnahme 110 ausgebildet ist, in der die Basis 10 eingesetzt wird. Die Aufnahme 110 weist auf ihrer Außenseite ein Außengewinde auf, das mit einem Innengewinde der Überwurfmutter 120 korrespondiert. Über die Überwurfmutter 120 wird die Basis 10 an dem Gehäuse 130 festgelegt. Über das Kabel 135 werden die Messwerte des nicht zu erkennenden Dehnmessstreifens 30 aus den Gehäusehälften 150 hinaus geführt und zu einer weiteren Steuerungseinheit übermittelt, über die dann beispielsweise Ventile gesteuert und Widerstände in Flexionsrichtung oder Extensionsrichtung eingestellt werden. Ebenfalls können Antriebe aktiviert und deaktiviert oder Energiespeicher aufgeladen und entladen werden, um eine aktive Verlagerung der Lageraufnahme 20 mit dem Lagerkörper 120 relativ zu einer zweiten Befestigungseinrichtung an dem Hydraulikaktuator 100 zu bewirken.

In dem dargestellten Ausführungsbeispiel ist die Linearkraftmesseinrichtung 1 an einem Gehäuse 130 des Hydraulikaktuators 100 angeordnet. Grundsätzlich besteht auch die Möglichkeit, dass eine solche Linearkraftmesseinrichtung 1 an einer Kolbenstange eines Hydraulikaktuators angeordnet ist.

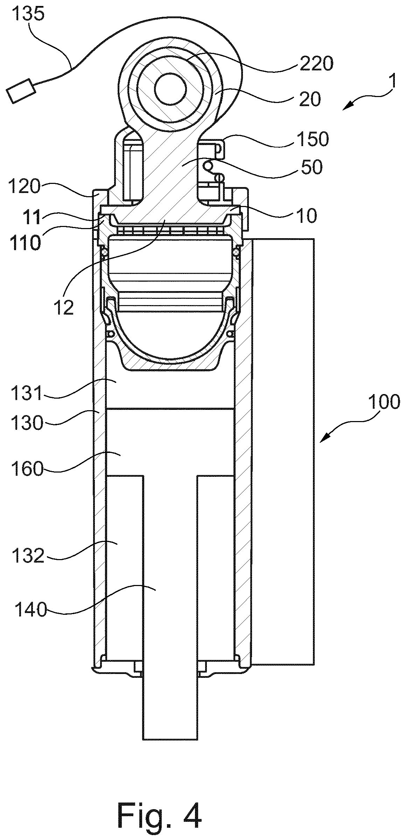

In der Figur 4 ist der Hydraulikaktuator 100 gemäß Figur 3 in einer Schnittdarstellung gezeigt. Innerhalb des Gehäuses 130 ist an einer Kolbenstange 140 ein Hydraulikkolben 160 längsbeweglich angeordnet und trennt eine Flexionskammer 131 von einer Extensionskammer 132. Die Aufnahme 110 an dem Gehäuse 130 ist ringförmig ausgebildet, so dass die Basis 10 mit dem ringförmigen Rand 11 auf der Aufnahme 110 aufliegt. Durch die Materialverringerung an dem Umfang der Basis 10, durch die der ringförmige Rand 11 gebildet wird, bildet sich ein Absatz 12 aus, der in Richtung auf das Gehäuse 10 vorsteht und somit eine Selbstzentrierung der Basis 10 in der Aufnahme 110 bewirkt. Das dem Kolben 160 abgewandte Ende der Kolbenstange 140 ist in dem montierten Zustand des Hydraulikaktuators 100 mit einer weiteren Befestigungseinrichtung versehen, um beispielsweise an einem Oberteil oder einem Unterteil eines Prothesengelenkes festgelegt zu werden. An der Kolbenstange 140, die aus dem Gehäuse 130 hinaussteht, kann ebenfalls eine Linearkraftmesseinrichtung 1 befestigt werden. Die Linearkraftmesseinrichtung 1 kann auch nur ausschließlich an der Kolbenstange 140 befestigt sein, während das Gehäuse 130 des Linearaktuators 100 ohne Linearkraftmesseinrichtung an einer orthopädietechnischen Einrichtung, beispielsweise Orthese, Prothese oder Rollstuhl, befestigt ist.

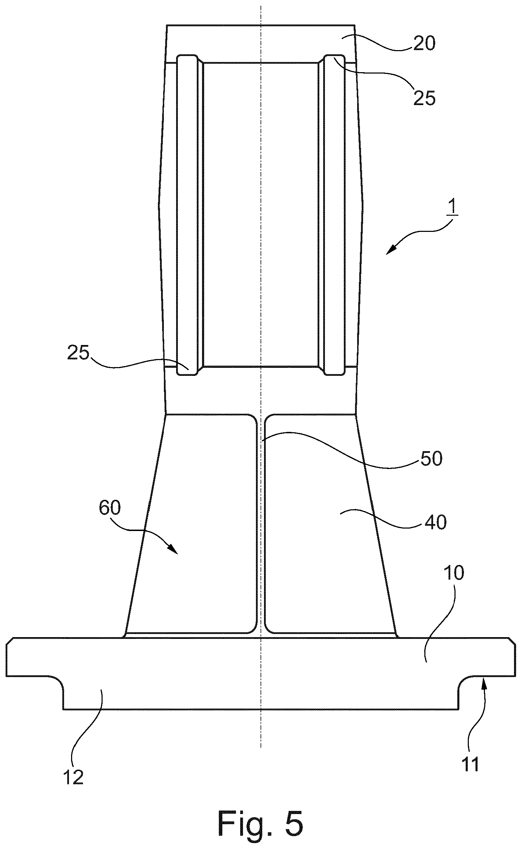

In der Figur 5 ist die Linearkraftmesseinrichtung gemäß Figur 1 in einem Vertikalschnitt dargestellt. In der Schnittdarstellung ist zu erkennen, dass sich die Basis 10 im Wesentlichen horizontal erstreckt, während sich das Versteifungselement 50 in Gestalt einer Wand senkrecht dazu in Vertikalrichtung erstreckt. Die Lageraufnahme 20 weist beabstandet von ihrer rechten und linken Stirnseite zwei Nuten 25 auf, die umlaufend ausgebildet sind und zur Aufnahme von nicht dargestellten Sicherungsringen dienen. Von der hülsenartigen Lageraufnahme 20 erstreckt sich in Richtung auf die Basis 10 neben der einstückig angeformten Wand als Versteifungselement 50 zwei Stege 40, von denen in der Schnittdarstellung nur ein Steg 40 zu erkennen ist. In der Figur 5 ist zu erkennen, dass der Steg 40 und damit auch der diesem gegenüberliegende, in der Schnittdarstellung nicht gezeigte zweite Steg 40 trapezförmig ausgebildet ist, das heißt, dass der Steg 40 in dem Bereich der Lageraufnahme 20 schmaler als in dem Bereich der Basis 10 ist. Durch die trapezförmige Ausgestaltung der Stege 40 kann eine erhöhte Stabilität der Linearkraftmesseinrichtung 1 gegen eine Verbiegung der Stege 40 bereitgestellt werden. Über die trapezförmige Ausgestaltung der Stege 40 ist es möglich, ein homogenes Dehnungsfeld in dem Bereich der Sensoren 30 oder Dehnmessstreifen zu erzeugen. Dadurch erhält man eine geringere Spannungskonzentration zwischen den Stegen 40 und der Basis 10.

In der Basis 10 ist im unteren Bereich ein ringförmiger Rand 11 ausgebildet, so dass sich nach unten hin ein Absatz 12 ausbildet, der von der Lageraufnahme 20 weg weist. Über diesen Absatz 12, der kreisförmig ausgebildet ist, kann eine Zentrierung in einer korrespondierend ausgebildeten Aufnahme 110 an dem Gehäuse des Hydraulikaktuators erfolgen. Die Hauptzentrierung erfolgt über die Überwurfmutter 120. Der Absatz 12 ist bevorzugt frei und nicht eingeklemmt, um das Sensorsignal nicht zu beeinflussen. Über ein nachgiebiges Element zwischen dem Absatz 12 und der Aufnahme 110 kann eine zusätzliche Zentrierungswirkung bereitgestellt werden. Das Versteifungselement 50 ist entlang der Wirkrichtung von Zugkräften und Druckkräften zwischen der Lageraufnahme 20 und der Basis 10 ausgerichtet. Zusammen mit der Lageraufnahme 20 und der Basis 10 sowie den Stegen 40 bildet das Versteifungselement 50 einen Aufnahmeraum 60 aus, in dem beispielsweise eine Steuerungselektronik untergebracht werden kann.

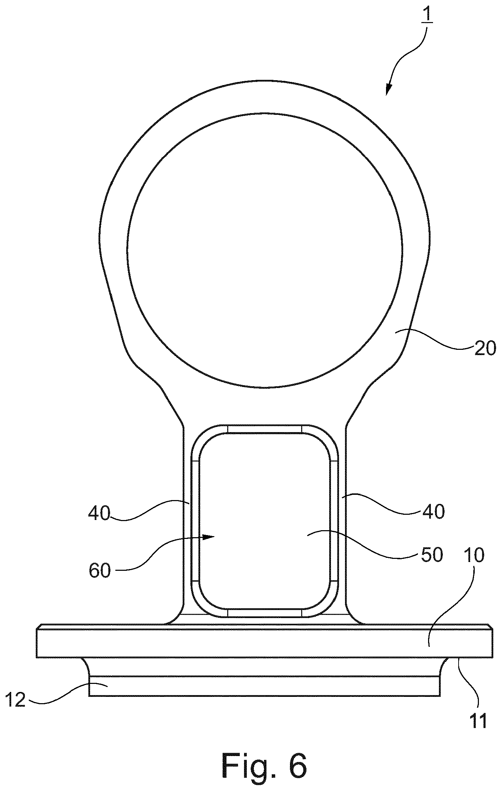

Figur 6 zeigt die Linearkraftmesseinrichtung 1 in einer Seitenansicht. In dieser Perspektive sind die parallele Ausrichtung der beiden Stege 40 sowie deren senkrechte Orientierung zu der Basis 10 zu erkennen. Das Versteifungselement 50 ist vollflächig zwischen den Stegen 40, der Lageraufnahme 20 und der Basis 10 ausgebildet, im dargestellten Ausführungsbeispiel einstückig ausgebildet, der Aufnahmeraum 60 weist eine angenähert rechteckige Kontur auf.

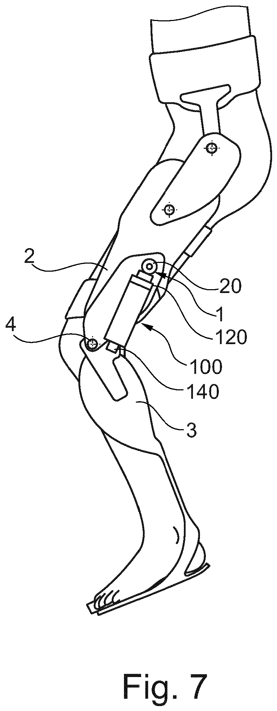

Figur 7 zeigt die Anbindung eines Hydraulikaktuators 100 an einer Orthese. In dem dargestellten Ausführungsbeispiel überbrückt die Orthese sämtliche Gelenke der unteren Extremität. Die Lageraufnahme 20 mit dem darin angeordneten Lagerkörper ist an einem Oberschenkelteil 2 befestigt, das über Befestigungsgurte an dem Oberschenkel festgelegt ist. Das Oberschenkelteil 2 ist proximal gelenkig mit einem Hüftgurt oder einer Hüftschale verbunden und distal über eine Schwenkachse 4 gelenkig mit einem Unterschenkelteil 3, das eine Fußabstützung aufweist. Das Unterteil 3 lässt sich um die Schwenkachse 4 in Extensionsrichtung und Flexionsrichtung relativ zu dem Oberschenkelteil 2 verschwenken. Der Hydraulikaktuator 100 ist sowohl an dem Oberschenkelteil 2 als auch an dem Unterschenkelteil 3 gelagert. An dem Unterschenkelteil 3 ist die Kolbenstange 140 befestigt. Schwenkbewegungen zwischen dem Oberschenkelteil 2 und dem Unterschenkelteil 3 werden durch den Hydraulikaktuator 100 unterstützt oder gedämpft. Die dabei auftretenden Kräfte werden über die Linearkraftmesseinrichtung 1 aufgenommen und an eine nicht dargestellte Steuerungseinrichtung übermittelt, um entweder Antriebe zu aktivieren oder zu deaktivieren oder um Dämpfungseinstellungen zu verändern.

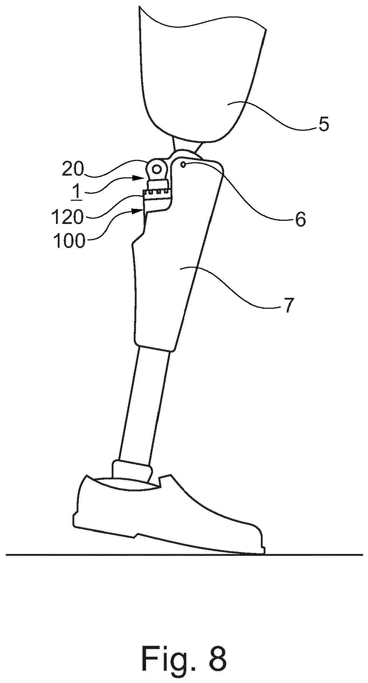

Ein alternatives Anwendungsbeispiel ist in der Figur 8 gezeigt, bei der der Hydraulikaktuator 100 zwischen einem Prothesenoberteil 5 in Gestalt eines Oberschenkelschaftes und einem Unterschenkelteil 7 angeordnet ist. Das Oberteil 5 und das Unterteil 7 sind in einem Prothesenkniegelenk um eine Schwenkachse 6 verschwenkbar miteinander verbunden. Die Lageraufnahme 20 der Linearkraftmesseinrichtung 1 ist an einem Ausleger an dem Oberteil 5 befestigt, während die nicht sichtbare Kolbenstange 140 an dem Unterschenkelteil 7 gelagert ist. Bei einer Flexion und bei einer Extension wird der Hydraulikaktuator 100 mit Linearkräften beaufschlagt, die über die Linearkraftmesseinrichtung gemessen werden. Auf Basis dieser Messwerte kann eine Steuerung des Hydraulikaktuators 100 erfolgen.

## Patentansprüche

1. Linearkraftmesseinrichtung für einen Hydraulikaktuator einer orthopädietechnischen Einrichtung mit einer Basis (10), einer Lageraufnahme (20) und zumindest einem Sensor (30) zur Erfassung von Längenänderungen zwischen der Basis (10) und der Lageraufnahme (20), wobei die Lageraufnahme (20) mit der Basis (10) über zwei einander gegenüberliegende Stege (40) stoffschlüssig verbunden und der zumindest eine Sensor (30) an einem der Stege (40) befestigt ist, wobei zwischen den Stegen (40) ein Versteifungselement (50) zur Versteifung der Stege (40) angeordnet ist und die Basis (10), die Lageraufnahme (20), die Stege (40) und das Versteifungselement (50) einen einseitig offenen Aufnahmeraum (60) ausbilden.

2. Linearkraftmesseinrichtung nach Anspruch 1, **dadurch gekennzeichnet, dass** die Stege (40) trapezförmig ausgebildet sind und sich in Richtung auf die Lageraufnahme (20) verjüngen.

3. Linearkraftmesseinrichtung nach einem der voranstehenden Ansprüche, **dadurch gekennzeichnet, dass** die Basis (10) als runde Scheibe ausgebildet ist.

4. Linearkraftmesseinrichtung nach einem der voranstehenden Ansprüche, **dadurch gekennzeichnet, dass** die Lageraufname (20) hülsenförmig mit einer runden Ausnehmung ausgebildet ist.

5. Linearkraftmesseinrichtung nach Anspruch 1, **dadurch gekennzeichnet, dass** der Sensor (30) mit einer elektronischen Auswerte- und/oder Steuerungseinrichtung (35) verbunden ist, die in dem Aufnahmeraum (60) angeordnet ist.

6. Linearkraftmesseinrichtung nach einem der voranstehenden Ansprüche, **dadurch gekennzeichnet, dass** der Sensor (30) an der Außenseite des Steges (40) festgelegt ist.

7. Hydraulikaktuator (100) für eine orthopädietechnische Einrichtung mit einer Kolben-Zylinder-Einheit und einer Aufnahme (110), an oder in der eine Linearkraftmesseinrichtung (1) nach einem der voranstehenden Ansprüche angeordnet ist.

8. Hydraulikaktuator nach Anspruch 7, **dadurch gekennzeichnet, dass** die Basis (10) vollflächig oder vollumfänglich auf der Aufnahme (110) aufliegt.

9. Hydraulikaktuator nach einem der Ansprüche 7 oder 8, **dadurch gekennzeichnet, dass** die Basis (10) drehbar auf oder in der Aufnahme (110) gelagert und klemmend fixiert ist.

10. Hydraulikaktuator nach Anspruch 9, **dadurch gekennzeichnet, dass** die Basis (10) über eine Überwurfmutter (120) an der Aufnahme (110) befestigt ist.

11. Hydraulikaktuator nach einem der Ansprüche 7 bis 10, **dadurch gekennzeichnet, dass** in der Lageraufnahme (20) ein Pendellager (220) oder ein Kugelgelenk angeordnet ist.

12. Hydraulikaktuator nach einem der Ansprüche 7 bis 11, **dadurch gekennzeichnet, dass** die Aufnahme (110) an einem Gehäuse (130) ausgebildet oder angeordnet ist.

## Claims

1. A linear force measurement device for a hydraulic actuator of an orthopedic device, having a base (10), a bearing receptacle (20), and at least one sensor (30) for detecting changes in length between the base (10) and the bearing receptacle (20), wherein the bearing receptacle (20) is connected to the base (10) in a material- locking manner via two mutually opposite webs (40), and the at least one sensor (30) is fastened to one of the webs (40), wherein a stiffening element (50) for stiffening the webs (40) is arranged between the webs (40), and the base (10), the bearing receptacle (20), the webs (40) and the stiffening element (50) form a receiving space (60) open at one side.

2. The linear force measurement device as claimed in claim 1, **characterized in that** the webs (40) are of trapezoidal shape and taper in the direction of the bearing receptacle (20).

3. The linear force measurement device as claimed in one of the preceding claims, **characterized in that** the base (10) is designed as a round disk.

4. The linear force measurement device as claimed in one of the preceding claims, **characterized in that** the bearing receptacle (20) is sleeve-shaped with a round recess.

5. The linear force measurement device as claimed in claim 1, **characterized in that** the sensor (30) is connected to an electronic evaluation and/or control device (35), which is arranged in the receiving space (60).

6. The linear force measurement device as claimed in one of the preceding claims, **characterized in that** the sensor (30) is secured to the outside of the web (40).

7. A hydraulic actuator (100) for an orthopedic device, having a piston-cylinder unit and a receptacle (110) at or in which a linear force measurement device (1) as claimed in one of the preceding claims is arranged.

8. The hydraulic actuator as claimed in claim 7, **characterized in that** the base (10) bears, with its complete surface area or its complete circumference, on the receptacle (110).

9. The hydraulic actuator as claimed in either of claims 7 and 8, **characterized in that** the base (10) is mounted rotatably on or in the receptacle (110) and is fixed by clamping.

10. The hydraulic actuator as claimed in claim 9, **characterized in that** the base (10) is fastened to the receptacle (110) via a union nut (120).

11. The hydraulic actuator as claimed in one of claims 7 through 10, **characterized in that** a self-aligning bearing (220) or a ball joint is arranged in the bearing receptacle (20).

12. The hydraulic actuator as claimed in one of claims 7 through 11, **characterized in that** the receptacle (110) is formed or arranged on a housing (130).

## Revendications

1. Dispositif de mesure de la force linéaire pour un actionneur hydraulique d'un dispositif orthopédique, comprenant une base (10), un logement de palier (20) et au moins un capteur (30) destiné à détecter des variations de longueur entre la base (10) et le logement de palier (20),
dans lequel
le logement de palier (20) est relié par coopération de matière à la base (10) par l'intermédiaire de deux entretoises (40) opposées l'une à l'autre, et ledit au moins un capteur (30) est fixé à l'une des entretoises (40),
un élément de rigidification (50) destiné à rigidifier les entretoises (40) est disposé entre les entretoises (40), et
la base (10), le logement de palier (20), les entretoises (40) et l'élément de rigidification (50) constituent un espace de réception (60) ouvert sur un côté.

2. Dispositif de mesure de la force linéaire selon la revendication 1, **caractérisé en ce que** les entretoises (40) sont de forme trapézoïdale et se rétrécissent en direction du logement de palier (20).

3. Dispositif de mesure de la force linéaire selon l'une des revendications précédentes,
**caractérisé en ce que** la base (10) est réalisée sous forme de disque rond.

4. Dispositif de mesure de la force linéaire selon l'une des revendications précédentes,
**caractérisé en ce que** le logement de palier (20) est réalisé en forme de douille avec un évidement rond.

5. Dispositif de mesure de la force linéaire selon la revendication 1,
**caractérisé en ce que** le capteur (30) est relié à un dispositif électronique d'évaluation et/ou de commande (35) disposé dans l'espace de réception (60).

6. Dispositif de mesure de la force linéaire selon l'une des revendications précédentes,
**caractérisé en ce que** le capteur (30) est immobilisé sur le côté extérieur de l'entretoise (40).

7. Actionneur hydraulique (100) pour un dispositif orthopédique comprenant une unité à piston et cylindre et un logement (110), sur ou dans lequel est disposé un dispositif de mesure de la force linéaire (1) selon l'une des revendications précédentes.

8. Actionneur hydraulique selon la revendication 7,
**caractérisé en ce que** la base (10) repose par toute sa surface ou par toute sa circonférence sur le logement (110).

9. Actionneur hydraulique selon l'une des revendications 7 ou 8,
**caractérisé en ce que** la base (10) est montée de façon mobile en rotation sur ou dans le logement (110) et est fixée par serrage.

10. Actionneur hydraulique selon la revendication 9,
**caractérisé en ce que** la base (10) est fixée au logement (110) par l'intermédiaire d'un écrou-raccord (120).

11. Actionneur hydraulique selon l'une des revendications 7 à 10,
**caractérisé en ce qu'**un palier à rotule (220) ou une rotule est disposé(e) dans le logement de palier (20).

12. Actionneur hydraulique selon l'une des revendications 7 à 11,
**caractérisé en ce que** le logement (110) est réalisé ou disposé sur un boîtier (130).
